(19)

Europäisches
Patentamt

European
Patent Office

Office européen
des brevets

(11)　**EP 4 600 241 A1**

(12)　**EUROPEAN PATENT APPLICATION**

published in accordance with Art. 153(4) EPC

(43) Date of publication:
**13.08.2025　Bulletin 2025/33**

(21) Application number: **23875189.5**

(22) Date of filing: **04.10.2023**

(51) International Patent Classification (IPC):
*C07C 67/297* (2006.01)　　*C07C 67/48* (2006.01)
*C07C 69/82* (2006.01)　　*C07B 61/00* (2006.01)
*C07B 63/00* (2006.01)

(52) Cooperative Patent Classification (CPC):
**C07B 61/00; C07B 63/00; C07C 67/08;**
**C07C 67/297; C07C 67/48; C07C 69/82**

(86) International application number:
**PCT/KR2023/015193**

(87) International publication number:
**WO 2024/076125 (11.04.2024 Gazette 2024/15)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **04.10.2022　KR 20220126487**

(71) Applicant: Hanwha Solutions Corporation
**Jung-gu**
**Seoul 04541 (KR)**

(72) Inventors:
• **KIM, Hyo Suk**
**Daejeon 34128 (KR)**
• **JUNG, Kitaeg**
**Daejeon 34128 (KR)**
• **KANG, Byungchan**
**Daejeon 34128 (KR)**
• **PARK, Seongmin**
**Daejeon 34128 (KR)**

(74) Representative: **Berggren Oy**
**P.O. Box 16**
**Eteläinen Rautatiekatu 10A**
**00101 Helsinki (FI)**

(54)　**METHOD FOR PREPARING ESTER-BASED COMPOSITION**

(57)　Provided is a method for preparing an ester-based composition using a continuous reaction system in which a plurality of reactors are connected in series, wherein the heat transfer performance, reactor productivity, and total reactor productivity can be improved by controlling operating conditions.

[FIG. 1]

**EP 4 600 241 A1**

**Description**

[Technical Field]

<u>Cross-Reference with Related Application(s)</u>

This application claims the benefit of priority to Korean Patent Application No. 10-2022-0126487, filed on October 4, 2022, the entire disclosure of which is incorporated herein by reference.

The present disclosure provides a method for preparing an ester-based composition using a continuous esterification reaction system in which a plurality of reactors are connected in series. In particular, it provides a method that can improve heat transfer performance, reactor productivity, and total reactor productivity in the manufacture of the ester-based composition.

[Background Technology]

Ester-based compounds produced by the esterification reaction of a polycarboxylic acid and an alcohol have been widely used as plasticizers.

The esterification reaction generally takes place at a high temperature in the presence of an esterification catalyst. Therefore, after the reaction is complete, the resulting reaction product includes the catalyst, by-products of catalyst decomposition, unreacted alcohol and unreacted polycarboxylic acid, monoesters, and reaction impurities. In order to obtain a pure ester-based compound, a post-treatment process such as neutralization, washing, alcohol removal, and/or filtration must be performed.

Accordingly, in order to obtain a highly pure ester-based compound, research has been actively conducted in various fields, such as methods for separating and purifying these by-products in the reaction product at high efficiency, methods for suppressing side reactions and catalyst decomposition during the esterification reaction, and methods for efficiently designing manufacturing facilities or processes.

Among the methods for suppressing side reactions and catalyst decomposition during the esterification reaction, there is a method of removing water produced during the esterification reaction by supplying heat to the reactor using a heat medium. This method uses a reaction system in which a plurality of reactors are connected in series. In such a system, the performance of heat supply to each reactor-i.e., the reactor's heat transfer performance-directly affects the esterification reaction rate. Consequently, when an ester-based compound is produced in a continuous process using a plurality of reactors with the same heat transfer performance, the reaction rate decreases in downstream reactors as the concentration of raw materials drops, and as a result, the amount of heat required to remove water also decreases. In other words, in downstream reactors, the heat transfer performance available exceeds the heat actually needed for the reaction, leaving spare heat transfer performance. This leads to a problem of reduced heat transfer performance, reactor productivity, and total reactor productivity in the production of the ester-based compound. Although various methods have been proposed to improve manufacturing efficiency and productivity of ester-based compounds by controlling reactor conditions, those methods have not yet provided sufficient improvements in terms of effectiveness, economic efficiency, and process feasibility.

[Detailed Description of the Invention]

[Technical Problem]

The present disclosure aims to provide a method for preparing an ester-based composition using a continuous esterification reaction system having a plurality of reactors connected in series, wherein heat transfer performance, reactor productivity, and total reactor productivity are all improved.

[Technical Solution]

According to the present disclosure, there is provided a method for preparing an ester-based composition using a continuous esterification reaction system in which a total of N reactors, from a first reactor through an Nth reactor, are connected in series, comprising the step of continuously feeding raw materials comprising a polycarboxylic acid and an alcohol into the continuous esterification reaction system to continuously produce a reaction product, and satisfying Mathematical equation 1 below:

[Mathematical equation 1]

$$P_1 < P_{N-1} \leq P_N$$

wherein Mathematical equation 1,
$P_1$ is a pressure (bar) of the first reactor,
$P_N$ is a pressure (bar) of the Nth reactor that follows the first reactor, and
N is an integer of 3 or greater.

[Advantageous Effects of the Invention]

In the method for preparing an ester-based composition according to the present disclosure, by performing a stepwise pressurization operation on the continuous esterification reaction system where a plurality of reactors are connected in series, it is possible to improve heat transfer performance, reactor productivity, and total reactor productivity at the same time.

[Brief Description of the Drawings]

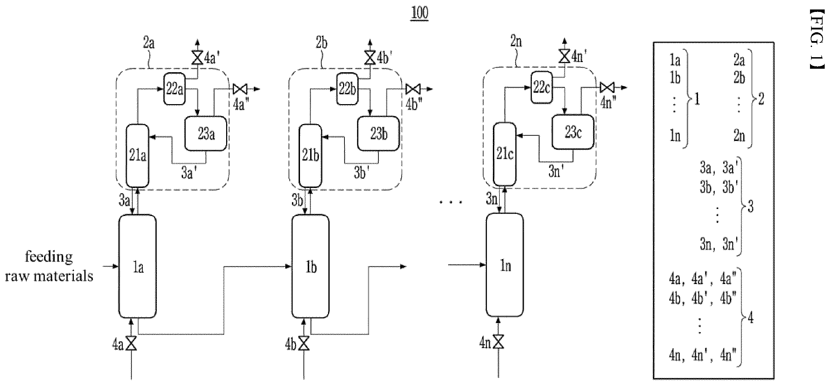

FIG. 1 is a schematic diagram illustrating one example of a continuous esterification reaction system that can be used in the method for preparing an ester-based composition according to the present disclosure.

[Explanation of Reference Numerals]

1 reaction unit
1a, 1b, 1n reactor
2 separation unit
2a, 2b, 2n separation device
3 recovery unit
3a, 3b, 3n unreacted alcohol first recovery device
3a', 3b', 3n' unreacted alcohol second recovery device
4 pressure control unit
4a, 4b, 4n first pressure control device
4a', 4b', 4n' second pressure control device
4a", 4b", 4n" third pressure control device
21a, 21b, 21n column separator
22a, 22b, 22n condenser
23a, 23b, 23n phase separator
100 continuous esterification reaction system

[Modes for Carrying Out the Invention]

In the present disclosure, terms such as "first" and "second" are used to describe various components. These terms are used only for the purpose of distinguishing one component from another.
Further, the terms used in this specification are used merely to illustrate exemplary embodiments, and are not intended to limit the present disclosure. Unless the context clearly indicates otherwise, the singular encompasses the plural. In this specification, terms such as "include," "comprise," and "have" indicate the presence of the stated feature, number, step, component, or combination thereof, and do not preclude the possibility of the presence or addition of one or more other features, numbers, steps, components, or combinations thereof.
Unless specifically mentioned otherwise, "%" or "part(s)" indicating content is based on weight.
The present disclosure may be modified in various ways and take various forms; therefore, certain embodiments will be presented below in detail by way of example. However, this is not intended to limit the invention to any particular disclosed form, and the invention should be understood as encompassing all modifications, equivalents, and substitutes that fall within the spirit and scope of the invention.
Below is a detailed description of the method for preparing an ester-based composition according to the present disclosure.

Conventionally, when an ester-based composition is produced using a continuous reaction system in which a plurality of reactors are connected in series, spare heat transfer performance remains in downstream reactors, resulting in reduced heat transfer performance, reactor productivity, and total reactor productivity.

In the present disclosure, in order to make maximum use of the spare heat transfer performance left in downstream reactors, the downstream reactors are pressurized relative to the upstream reactors in a continuous esterification reaction system, thereby increasing reaction temperature and reactivity, and consequently improving productivity in each reactor.

Specifically, the method for preparing an ester-based composition according to the present disclosure is a method of producing an ester-based composition using a continuous esterification reaction system in which a total of N reactors from a first reactor to an Nth reactor are connected in series, comprising the step of continuously feeding raw materials including a polycarboxylic acid and an alcohol into the continuous esterification reaction system to continuously produce a reaction product, and satisfying Mathematical equation 1 below:

$$[\text{Mathematical equation 1}]$$

$$P_1 < P_{N-1} \leq P_N$$

wherein Mathematical equation 1,
$P_1$ is a pressure (bar) of the first reactor,
$P_N$ is a pressure (bar) of the Nth reactor that follows the first reactor, and
N is an integer of 3 or greater.

When downstream reactors are pressurized, the temperature within each pressurized reactor increases, resulting in a rise in the average temperature throughout the reactors. This rise in the average reactor temperature increases the esterification reaction rate, and thus increases total reactor productivity.

Moreover, if the increase in pressure in the downstream reactors, relative to the first reactor, exceeds a certain level, the above effects are further enhanced. However, if the pressure in downstream reactors is excessively high, the reaction temperature inside the reactors may increase significantly such that the amount of heat required exceeds the reactor's heat transfer performance, and the content of heat residues may increase. As a result, side reactions and catalyst decomposition reactions may occur, and reactivity may actually decrease. Thus, it is preferable to control the pressure increase in the downstream reactors to or below a certain level.

Specifically, the method may further satisfy Mathematical equation 2 below:

$$[\text{Mathematical equation 2}]$$

$$P_1 + 0.01 \leq P_N \leq P_1 + 2$$

wherein Mathematical equation 2, $P_1$, $P_N$, and N are as defined above.

More specifically, the method may further satisfy any one of Mathematical equations 2-1 to 2-5 below:

$$[\text{Mathematical equation 2-1}]$$

$$P_1 + 0.02 \leq P_N \leq P_1 + 2$$

$$[\text{Mathematical equation 2-2}]$$

$$P_1 + 0.05 \leq P_N \leq P_1 + 1.5$$

$$[\text{Mathematical equation 2-3}]$$

$$P_1 + 0.06 \leq P_N \leq P_1 + 1.5$$

[Mathematical equation 2-4]

$$P_1 + 0.1 \leq P_N \leq P_1 + 1$$

[Mathematical equation 2-5]

$$P_1 + 0.1 \leq P_N \leq P_1 + 0.5$$

wherein Mathematical equations 2-1 to 2-5, $P_1$, $P_N$, and N are as defined above.

In addition, in Mathematical equations 1 and 2, the pressure ($P_1$) of the first reactor may be from 1 bar to 4 bar. More specifically, $P_1$ may be at least 1 bar, or 1.1 bar, or 1.2 bar, or 1.24 bar, and up to 4 bar, or 3 bar, or 2 bar, or 1.5 bar, or 1.3 bar, or 1.28 bar, or 1.26 bar. When P1 is controlled within this range, process feasibility and reactor productivity are superior.

Further, in Mathematical equations 1 and 2, N is the number of reactors in the reaction system. Specifically, N is 3 or more, or 4 or more, and 20 or less, or 10 or less, or 8 or less, or 5 or less, expressed as an integer.

In the method according to the present disclosure, the pressure in each reactor can be adjusted by controlling the amount of inert gas introduced into the reactor so that the above pressure conditions are satisfied. Additionally, pressure inside each reactor can be controlled by adjusting the amount of uncondensed gas including inert gas, and water that are discharged as a result of the reaction.

For example, increasing the amount of inert gas introduced into a reactor or decreasing the amount of uncondensed gas and water being discharged can raise the pressure inside that reactor.

The amount of inert gas, uncondensed gas, and water can be adjusted through a pressure control device of the pressure control unit provided in the continuous esterification reaction system. Detailed explanations of this are provided below in the description of the continuous esterification reaction system.

Meanwhile, in the method according to the present disclosure, the raw materials for producing the ester-based composition are prepared by mixing a polycarboxylic acid and an alcohol. Accordingly, the method of the present disclosure may further include a step of mixing the polycarboxylic acid and the alcohol prior to feeding the raw materials.

The mixing of the polycarboxylic acid and the alcohol can be performed by a conventional method. Before feeding them into the reactor, a mixer or other mixing device can be used to homogenize them, preventing uneven esterification reactions according to different positions inside the reactor.

In the raw materials, the polycarboxylic acid may be an aliphatic or aromatic carboxylic acid with carboxyl (-COOH) groups of 2 or more, specifically 2 to 4 in the molecule, or an anhydride of that polycarboxylic acid.

Specifically, the polycarboxylic acid may be an aliphatic carboxylic acid having 2 to 20 carbon atoms or an aromatic carboxylic acid having 6 to 20 carbon atoms. For example, the polycarboxylic acid may be at least one selected from the group consisting of adipic acid, azelaic acid, phthalic acid, isophthalic acid, terephthalic acid, citric acid, trimellitic acid, and their anhydrides, but is not limited thereto. Preferably, the polycarboxylic acid may be at least one selected from the group consisting of phthalic acid, isophthalic acid, terephthalic acid, and their anhydrides and derivatives, and more preferably, terephthalic acid or its anhydride.

The alcohol may be a chain or branched aliphatic alcohol having 1 to 20 carbon atoms, or 4 to 20 carbon atoms, or 5 to 15 carbon atoms. Specifically, it may be at least one selected from the group consisting of butanol, hexanol, 2-ethylhexanol, isononyl alcohol, isodecyl alcohol, and propylheptanol.

In one example, the method for producing an ester-based composition of the present disclosure may be a method of producing dioctyl terephthalate (di-2-ethylhexyl terephthalate, DOTP) using terephthalic acid as the polycarboxylic acid and 2-ethylhexanol as the alcohol.

Additionally, when preparing the raw materials, a catalyst for esterification reaction may also be fed.

The catalyst for esterification reaction may be fed into the mixture of the polycarboxylic acid and the alcohol or into each of the polycarboxylic acid or the alcohol before those are mixed. The catalyst for esterification reaction may also be fed directly into the reactor.

Examples of the catalyst for esterification reaction include organometallic catalysts, organosulfonic acids, acid catalysts, or mixtures thereof. Specifically, examples include tetraalkyltitanates such as tetraisopropyl titanate, tetra n-butyl titanate (TnBT), tetraoctyl titanate, butyl tin maleate, and so on as the organometallic catalysts; organosulfonic acids such as para-toluenesulfonic acid, methanesulfonic acid, ethanesulfonic acid, propanesulfonic acid, or butanesulfonic acid; and acid catalysts such as formic acid, nitric acid, acetic acid, hydrochloric acid, phosphoric acid, and sulfuric acid. Any one of these or a mixture of two or more, may be used.

The amounts of the polycarboxylic acid, the alcohol, and the catalyst for esterification reaction used in the present disclosure are not particularly limited, and can be suitably determined based on the properties and use of the ester-based composition to be produced.

In one example, in a method of producing dioctyl terephthalate by using terephthalic acid and 2-ethylhexanol, the polycarboxylic acid corresponding to terephthalic acid and the alcohol corresponding to 2-ethylhexanol may be fed at a molar ratio of 1:2 to 1:4, or 1:3 to 1:3.5.

Also, the catalyst may be fed in an amount of about 100 to about 2000 ppm based on the total weight of the alcohol, more specifically 100 ppm or more, or 200 ppm or more, or 300 ppm or more, or 500 ppm or more, and 2000 ppm or less, or 1500 ppm or less, or 1000 ppm or less, or 700 ppm or less. When fed in the above range, maximum reaction efficiency is achieved relative to the amount fed.

The reaction conditions for the esterification of the polycarboxylic acid and the alcohol are also not particularly limited but, for example, it may be carried out at a temperature of 180°C to 260°C.

When the raw materials including the polycarboxylic acid and the alcohol, optionally including an esterification catalyst, are continuously fed into the first reactor of the continuous esterification reaction system, the esterification reaction takes place in that first reactor. The resulting reaction product is sequentially transferred to each subsequent reactor in series after the first reactor. When the reaction product is introduced into a subsequent reactor, the esterification reaction occurs in the same way as in the first reactor.

Because the reacting raw materials pass continuously through a plurality of reactors connected in series, it is possible to optimize the entire process by independently adjusting process variables at each reactor, thereby maximizing the efficiency of the resulting manufacturing process.

Specifically, in the method according to the present disclosure, by controlling the pressurization conditions of each reactor so as to satisfy the conditions described above, using the pressure control unit provided in the continuous esterification reaction system, it becomes possible to make full use of the heat transfer performance of each reactor and to improve both reactor productivity and total reactor productivity.

Meanwhile, the continuous esterification reaction system used in the method according to the present disclosure has a structure in which a total of N reactors from the first reactor to the Nth reactor are connected in series.

FIG. 1 is a schematic diagram illustrating one example of the continuous esterification reaction system that can be used in the method for preparing an ester-based composition according to the present disclosure. FIG. 1 is merely an example for explaining the present disclosure; it is not intended to limit the present disclosure.

Referring to FIG. 1, the continuous esterification reaction system (10) includes: a reaction unit (1) in which N reactors connected in series conduct an esterification reaction on raw materials including a polycarboxylic acid and an alcohol; a separation unit (2) that comprises a separation device separating unreacted alcohol from the reaction product introduced from a reactor of the reaction unit; a recovery unit (3) that comprises a recovery device feeding the unreacted alcohol separated in the separation device of the separation unit back into the reactor of the reaction unit; and a pressure control unit (4) that comprises a pressure control device controlling the pressure inside the reactor of the reaction unit. Here, N is an integer of 3 or more, more specifically 3 or more, or 4 or more, and 20 or less, or 10 or less, or 8 or less, or 5 or less.

In the continuous esterification reaction system, the reaction unit (1) includes N reactors (1a, 1b, ... 1n, where "n" is an alphabetic label corresponding to the integer N) connected in series in such a manner that the esterification reaction proceeds in sequence. Therefore, when the raw materials including the polycarboxylic acid and the alcohol, and optionally an esterification catalyst, are continuously fed to the first reactor (1a) of the reaction unit (1), the esterification reaction occurs in the first reactor (1a). The resulting reaction product is then transferred sequentially to each subsequent reactor connected in series, specifically the second reactor (1b) through the Nth reactor (1n). The reaction product is transferred through a transfer line, such as piping, that connects each pair of reactors. For example, as shown in FIG. 1, the first reactor and the second reactor are connected by a reaction product transfer line located at the bottom of the first reactor, through which the reaction product in the first reactor is transferred. When the reaction product flows into the subsequent reactor, the esterification reaction proceeds therein in the same manner as in the first reactor.

In addition, an inert gas is fed into the lower part of each reactor in the reaction unit to control the pressure inside that reactor. Besides serving as a means for adjusting reactor pressure, the inert gas can also inhibit side reactions by creating an inert atmosphere during the esterification reaction inside the reactor.

The inert gas is fed into the interior of the reactor through an inert gas inlet line connected to the lower part of the reactor, and is discharged to the separation unit along with the reaction product (reaction by-products) through the reaction product discharge line connected to the upper portion of the reactor.

Nitrogen or the like may be used as the inert gas.

Also, the inert gas is supplied in an amount that satisfies the pressure conditions inside the reactor, and the amount supplied can be controlled by the pressure control device described below.

Further, in the continuous esterification reaction system, the separation unit (2) is connected to the reaction unit (1), more specifically to the reactor of the reaction unit, and includes separation devices (2a, 2b, 2n) for separating unreacted alcohol from the reaction product flowing from the reactor. Specifically, each separation device (2a, 2b, 2n) may include: a column separator (21a, 21b, 21n) in which vapor-liquid separation is carried out on the reaction product introduced from the reactor of the reaction unit; a condenser (22a, 22b, 22n) that condenses the gaseous substance separated and discharged in the column separator, converting it to a liquid phase, while discharging uncondensed gas (including inert gas); and a phase separator (23a, 23b, 23n) that separates, into an organic layer and an aqueous layer, the substance converted to the liquid phase in the condenser.

In the separation unit, each separation device may be connected on a one-to-one basis for each reactor in the reaction unit, or one separation device may be connected to two or more reactors. For example, if there are five reactors in the reaction unit, one separation device may be connected to each of the first through third reactors, and a second separation device may be connected to the fourth and fifth reactors. Accordingly, if there are N reactors, the separation unit may include between 1 and N separation devices, where N is as defined above.

Furthermore, in the continuous esterification reaction system, the recovery unit (3) includes an unreacted alcohol first recovery device (3a, 3b, 3n) that collects the liquid including unreacted alcohol separated by vapor-liquid separation in the column separator (21a, 21b, 21n) of the separation device in the separation unit (2) and then feeds it back to the reactor (1a, 1b, 1n), and may further include an unreacted alcohol second recovery device (3a', 3b', 3n') that collects the organic layer including the unreacted alcohol separated by the phase separator (23a, 23b, 23n) and feeds it back to the top of the column separator (21a, 21b, 21n). Each unreacted alcohol first or second recovery device may take various forms such as a storage tank or a recovery line.

Additionally, in the continuous esterification reaction system, the pressure control unit (4) controls the pressure inside each reactor of the reaction unit so that the above pressure conditions are satisfied.

Specifically, the pressure control unit may include a first pressure control device (4a, 4b, 4n) located in the inert gas inlet line connected to the lower part of each reactor, which adjusts the pressure in the reactor by controlling the amount of inert gas continuously flowing into the lower part of each reactor. In addition, the pressure control unit may include a second pressure control device (4a', 4b', 4n') located in the uncondensed gas discharge line, which is connected to the top of the condenser in the separation device of the separation unit, and that adjusts the reactor pressure by controlling the amount of uncondensed gas discharged from the separation unit. Moreover, the pressure control unit (4) may include a third pressure control device (4a", 4b", 4n") located in the discharge line of the aqueous layer separated by gravity separation in the phase separator (23a), which adjusts the reactor pressure by controlling the amount of water discharged. Therefore, the column separator, condenser, and phase separator in the separation unit connected to each reactor have the same pressure as that reactor.

Each pressure control device in the pressure control unit (4) may be, for example, a control valve, although it is not limited thereto.

In the continuous esterification reaction system having the structure described above, when the esterification reaction takes place in the first reactor (1a) of the reaction unit, an ester compound is generated as a result. Besides the ester compound, this reaction product also includes unreacted raw materials and water. As the reaction product, which includes the ester compound and the unreacted raw materials, is transferred to each subsequent reactor in series after the first reactor (1a), for instance, the second reactor (1b), the esterification reaction proceeds repeatedly. As a result, as the esterification reaction progresses through these subsequent reactors, the content of the ester compound in the reaction product increases, while the content of unreacted raw materials decreases, and in the final reactor, only the ester compound remains. Meanwhile, the reaction product that includes unreacted raw materials such as unreacted alcohol and water is discharged to the first separation device (2a) through the reaction product discharge line at the top of the first reactor (1a), under heat and pressure inside the first reactor (1a). When the reaction product is introduced into the column separator (21a) in the first separation device (2a) via the reaction product discharge line, vapor-liquid separation takes place in the column separator, producing a liquid that is separated and returned to the first reactor (1a) through a recovery line (3a) serving as the recovery device at the bottom of the column separator. Gaseous substance is discharged to the condenser (22a) through the discharge line at the top of the column separator (21a). The liquid primarily includes unreacted alcohol and may also include a small amount of low-boiling ester compounds that have escaped from the reactor in a vapor state. Accordingly, the liquid that is fed back to the first reactor can be reused in the esterification reaction. The gaseous substance may further include alcohol that has not condensed and water. In the condenser (22a), the gaseous substance discharged from the column separator (21a) is cooled into a liquid phase, while the uncondensed gas, which includes inert gas, is discharged. The liquid substance condensed by the condenser (22a) is sent through the condenser's discharge line to the phase separator (23a). In the phase separator (23a), the liquid substance that was introduced from the condenser (22a) is separated by gravity into an organic layer and an aqueous layer. The separated organic layer, which primarily includes unreacted alcohol, is fed back to the top of the column separator (21a) via the recovery line (3a'), and the aqueous layer is discharged externally.

In addition, the continuous esterification reaction system may further include one or more neutralization vessels for neutralizing the ester-based composition produced through the reactors, and one or more purification tanks for further refining the composition. The neutralization vessel or purification tank may be connected to the final (nth) reactor among the series-connected reactors.

In one example, if a purification tank is additionally included, once the esterification reaction in the reaction unit is complete, and the reaction product including the ester compound from the final reactor flows into the neutralization vessel or purification tank, unreacted alcohol that remains in the reaction product can be separated and removed, producing a purified ester compound.

The purification tank may include a separation column or a flash vessel. If it includes a separation column, the final composition ratio of the produced composition can vary depending on the number of stages in the separation column. Therefore, it is preferable to determine the number of separation column stages in consideration of the composition ratio and characteristics of the composition to be manufactured. If a flash vessel is included, it is preferable to operate under vacuum conditions in order to efficiently remove unreacted alcohol from the hot reaction product.

Moreover, in the purification tank, the unreacted alcohol that is separated may be fed back to one or more of the reactors in the reaction unit and reused for the continuous esterification reaction.

Additionally, the continuous esterification reaction system may further include devices typically used in process design, such as a mixing apparatus for mixing the polycarboxylic acid and the alcohol, a decanter, a heat exchanger, a reboiler, and a pump. These devices may be disposed as appropriate for their intended uses.

As described above, in the method for preparing an ester-based composition according to the present disclosure, by continuously feeding raw materials including a polycarboxylic acid and an alcohol into the continuous esterification reaction system, a reaction product can be continuously produced, while at the same time, by controlling the pressurization conditions in each reactor through the pressure control unit included in the reaction system so as to satisfy the conditions described above, the heat transfer performance of each reactor can be utilized to the maximum extent, and both reactor productivity and total reactor productivity can be improved.

Below, preferred Examples are presented to aid understanding of the present disclosure. However, these Examples are provided only for easier comprehension of the present disclosure, and do not limit its subject matter in any way.

In the following Examples and Comparative Examples, simulations were conducted using the Aspen Plus process simulation program for a continuous esterification reaction system in which four reactors are connected in series.

**Example 1**

As shown in FIG. 1, a continuous reaction system configured with four reactors connected in series was applied to produce an ester-based composition. As the reaction raw materials, terephthalic acid and 2-ethylhexanol were used in a molar ratio of 1:3.4. Tetra n-butyl titanate (TnBT) was used as the catalyst for esterification reaction, in an amount of 500 ppm relative to the total weight of 2-ethylhexanol. When these reaction raw materials were fed into the first of the series-connected reactors, the resulting reaction product was sequentially sent on to subsequent reactors. The pressure in each reactor was set to the conditions shown in Table 1 below by controlling the amounts of nitrogen gas fed into each reactor through the pressure control device, the amount of uncondensed gas discharged from the top of the condenser, and the amount of water discharged after gravity separation in the phase separator.

**Examples 2 to 4 and Comparative Examples 1 to 3**

Except for setting the pressure in each reactor to the conditions shown in Table 1 below, the ester-based composition was produced in the same manner as in Example 1.

**Experimental Examples**

In producing the ester-based composition according to the above Examples and Comparative Examples, the increase in the average reactor temperature, heat transfer performance, reactor productivity, and total reactor productivity were evaluated as follows.

(1) Increase in average reactor temperature (unit: °C)

From the simulation results obtained using the Aspen Plus process simulation program, the temperature of each reactor was derived for the production of the ester-based composition in each Example or Comparative Example. The temperature values of all reactors were summed and then divided by the number of reactors to obtain the average reactor temperature (°C). The increase in the average reactor temperature was calculated according to Mathematical equation 3 below:

[Mathematical equation 3]

$$(\text{Increase in average reactor temperature, °C}) = Ta - Tb$$

wherein Mathematical equation 3,

Ta is the average reactor temperature (°C) in each Example or Comparative Example, obtained by summing the temperatures of all reactors (as derived via the Aspen Plus simulation) and dividing by the total number of reactors, and

Tb is the average reactor temperature (°C) in Comparative Example 1, obtained by summing the temperatures of all reactors (as derived via the Aspen Plus simulation for Comparative Example 1) and dividing by the total number of reactors.

(2) Ratio of heat transfer performance (%) for each reactor

According to Mathematical equation 4 below, the heat transfer performance (MJ/h·°C·m$^3$) of each reactor in the Examples and Comparative Examples was determined, and the heat transfer performance ratio for each reactor was calculated as a percentage relative to the heat transfer performance of the first reactor (n=1, reactor #1).

[Mathematical equation 4]

$$(\text{Reactor heat transfer performance, MJ/h·°C·m}^3) = A1 / (A2 \times A3)$$

wherein Mathematical equation 4,

A1 is the thermal energy Q (MJ/h, megajoule per hour) transferred from the heat supply utility to the fluid inside the reactor,

A2 is the log mean temperature difference (LMTD, °C) between the heat supply utility and the reactor, and

A3 is the reactor liquid volume (m$^3$).

(3) Ratio of reactor productivity (%) for each reactor

According to Mathematical equation 5 below, the productivity (kg/hr·m$^3$) of each reactor was calculated, and the reactor productivity ratio (%) of each reactor was determined by expressing its productivity as a percentage relative to that of the first reactor (n=1, reactor #1).

[Mathematical equation 5]

$$(\text{Reactor productivity, kg/hr·m}^3) = P / V$$

wherein Mathematical equation 5,

P is the quantity (kg/hr) of reaction product produced in each reactor, as derived from the simulation using the Aspen Plus process simulation program, and
V is the liquid volume (m$^3$) of the reactor.

(4) Average reactor productivity (%) and total reactor productivity (%)

After summing the reactor productivity ratios of all reactors calculated in (3) above, that total was divided by the number of reactors to calculate the average reactor productivity as a percentage. The average reactor productivity is then expressed as a percentage relative to the average reactor productivity of Comparative Example 1.

**[Table 1]**

| | | Example 1 | Example 2 | Example 3 | Example 4 | Comparative Example 1 | Comparative Example 2 | Comparative Example 3 |
|---|---|---|---|---|---|---|---|---|
| Operating pressure in reactor (bar) | Reactor #1(P1) | 1.24 | 1.24 | 1.24 | 1.24 | 1.24 | 1.24 | 1.24 |
| | Reactor #2(P2) | 1.30 | 1.35 | 1.35 | 1.35 | 1.24 | 1.22 | 1.35 |
| | Reactor #3(P3) | 1.30 | 1.35 | 1.42 | 1.42 | 1.24 | 1.20 | 1.24 |
| | Reactor #4(P4) | 1.30 | 1.35 | 1.42 | 1.50 | 1.24 | 1.18 | 1.24 |
| Increase in average reactor temperature (°C) | | 1.9 | 3.3 | 4.6 | 5.3 | 0.0 | -1.3 | 1.1 |
| Ratio of heat transfer performance (%) (based on reactor #1) | Reactor #1 | 100% | 100% | 100% | 100% | 100% | 100% | 100% |
| | Reactor #2 | 87% | 96% | 96% | 96% | 69% | 65% | 89% |
| | Reactor #3 | 69% | 80% | 95% | 95% | 64% | 55% | 58% |
| | Reactor #4 | 63% | 61% | 73% | 95% | 55% | 47% | 51% |
| Ratio of reactor productivity (%) (based on reactor#1) | Reactor #1 | 100% | 100% | 100% | 100% | 100% | 100% | 100% |
| | Reactor #2 | 89% | 95% | 95% | 95% | 82% | 79% | 94% |
| | Reactor #3 | 80% | 85% | 93% | 93% | 74% | 70% | 73% |
| | Reactor #4 | 75% | 79% | 87% | 98% | 68% | 62% | 67% |
| Average reactor productivity (%) | | 86 | 89.75 | 93.75 | 96.5 | 81 | 75.5 | 83.5 |
| Total reactor productivity (%) (based on Comparative Example 1) | | 106 | 111 | 116 | 120 | 100 | 93 | 103 |

In Examples 1 and 2, in a continuous esterification reaction system in which four reactors are connected in series, the pressure in the downstream reactors is increased to a certain level relative to the first reactor, and all downstream reactors have the same increased pressure. In Examples 3 and 4, the reaction pressure is sequentially increased from the first reactor to the last reactor.

On the other hand, in Comparative Example 1, in the continuous esterification reaction system with the four reactors connected in series, all reactors have the same pressure; in Comparative Example 2, the reaction pressure is sequentially decreased; and in Comparative Example 3, the pressure is raised in the second reactor relative to the first reactor, but returned to the same pressure as the first reactor for the subsequent reactors.

In Comparative Example 1, the heat transfer performance of the second through fourth reactors is 55% to 69%, except for the first reactor, indicating that spare heat transfer performance remained. In contrast, in Examples 1 to 4, where the pressure of the downstream reactors is increased, each reactor's heat transfer performance increases, and productivity also increases. This improvement grows more pronounced as the pressure increase becomes larger. Moreover, in Example 5, in which pressure continues to increase from one reactor to the next, each reactor's heat transfer performance is used at nearly 100%, and as a result, total reactor productivity is the highest.

Meanwhile, in Comparative Example 2, in which reactor pressure decreases toward the downstream reactors, both the heat transfer performance ratio and productivity at each stage are reduced compared to Comparative Example 1. In Comparative Example 3, the second reactor, whose pressure is raised relative to the first reactor, exhibits enhanced heat transfer performance and productivity, but these values decrease again in the third and fourth reactors, whose pressures are lowered back to that of the first reactor.

From these results, it can be seen that performing a stepwise pressurization operation on a continuous esterification reaction system in which a plurality of reactors are connected in series can improve the heat transfer performance of each reactor, as well as reactor productivity and total reactor productivity.

**Claims**

1.  A method for preparing an ester-based composition using a continuous esterification reaction system in which a total of N reactors, from a first reactor through an Nth reactor, are connected in series, comprising the step of continuously feeding raw materials comprising a polycarboxylic acid and an alcohol into the continuous esterification reaction system to continuously produce a reaction product, and satisfying Mathematical equation 1 below:

$$[\text{Mathematical equation 1}]$$

$$P_1 < P_{N-1} \leq P_N$$

    wherein Mathematical equation 1,

    $P_1$ is a pressure (bar) of the first reactor,
    $P_N$ is a pressure (bar) of the Nth reactor that follows the first reactor, and N is an integer of 3 or greater.

2.  The method for preparing an ester-based composition of Claim 1, which satisfies Mathematical equation 2 below:

$$[\text{Mathematical equation 2}]$$

$$P_1 + 0.01 \leq P_N \leq P_1 + 2$$

    wherein Mathematical equation 2, $P_1$, $P_N$, and N are as defined in Claim 1.

3.  The method for preparing an ester-based composition of Claim 2, wherein $P_1$ is from 1 bar to 4 bar.

4.  The method for preparing an ester-based composition of Claim 1, wherein N is an integer from 3 to 20.

5.  The method for preparing an ester-based composition of Claim 1, wherein the polycarboxylic acid is at least one selected from the group consisting of phthalic acid, isophthalic acid, terephthalic acid, an anhydride thereof, and a derivative thereof.

6.  The method for preparing an ester-based composition of Claim 1, wherein the alcohol is an aliphatic alcohol having 1 to 20 carbon atoms.

7.  The method for preparing an ester-based composition of Claim 1, wherein the raw materials further comprise a catalyst for esterification reaction.

8.  The method for preparing an ester-based composition of Claim 1, wherein the continuous esterification reaction system comprises:

    a reaction unit in which N reactors, carrying out the esterification reaction of raw materials comprising the polycarboxylic acid and the alcohol, are connected in series;
    a separation unit comprising a separation device that separates unreacted alcohol from the reaction product flowing in from the reactor of the reaction unit;
    a recovery unit comprising a recovery device that feeds the unreacted alcohol separated in the separation device of the separation unit back into the reactor of the reaction unit; and
a pressure control unit that comprises a pressure control device for adjusting the pressure in the reactor of the reaction unit.

9. The method for preparing an ester-based composition of Claim 8, wherein the separation device comprises:

a column separator in which vapor-liquid separation is carried out on the reaction product flowing in from the reactor of the reaction unit;
a condenser that condenses the gaseous substance separated and discharged as a result of vapor-liquid separation in the column separator into a liquid phase, while discharging uncondensed gas; and
a phase separator that separates the substance converted into a liquid phase in the condenser into an organic layer and an aqueous layer.

【FIG. 1】

## INTERNATIONAL SEARCH REPORT

International application No.

**PCT/KR2023/015193**

| A. | CLASSIFICATION OF SUBJECT MATTER |
|---|---|

**C07C 67/297**(2006.01)i; **C07C 67/48**(2006.01)i; **C07C 69/82**(2006.01)i; **C07B 61/00**(2006.01)i; **C07B 63/00**(2006.01)i

According to International Patent Classification (IPC) or to both national classification and IPC

| B. | FIELDS SEARCHED |
|---|---|

Minimum documentation searched (classification system followed by classification symbols)

C07C 67/297(2006.01); B01J 31/02(2006.01); B01J 31/12(2006.01); C07C 67/08(2006.01); C07C 69/82(2006.01); C08G 63/12(2006.01); C08G 63/78(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 에스테르계 조성물(ester-based composition), 연속식 에스테르화(continuous esterification), 반응기(reactor), 압력(pressure)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | KR 10-2022-0067352 A (LG CHEM, LTD.) 24 May 2022 (2022-05-24)<br>See claim 1. | 1-9 |
| A | KR 10-1663586 B1 (AEKYUNG CHEMICAL CO., LTD.) 10 October 2016 (2016-10-10)<br>See entire document. | 1-9 |
| A | KR 10-2022-0041009 A (LG CHEM, LTD.) 31 March 2022 (2022-03-31)<br>See entire document. | 1-9 |
| A | KR 10-2014-0071536 A (SAMSUNG ELECTRONICS CO., LTD.) 12 June 2014 (2014-06-12)<br>See entire document. | 1-9 |
| A | US 2010-0137631 A1 (DE MUNCK, Nicolaas Anthony et al.) 03 June 2010 (2010-06-03)<br>See entire document. | 1-9 |

☐ Further documents are listed in the continuation of Box C.  ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **09 January 2024** | **10 January 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT
### Information on patent family members

International application No.

**PCT/KR2023/015193**

| Patent document cited in search report | | | Publication date (day/month/year) | Patent family member(s) | | | Publication date (day/month/year) |
|---|---|---|---|---|---|---|---|
| KR | 10-2022-0067352 | A | 24 May 2022 | CN | 114829332 | A | 29 July 2022 |
| | | | | EP | 4023628 | A1 | 06 July 2022 |
| | | | | EP | 4023628 | A4 | 20 September 2023 |
| | | | | JP | 2023-507538 | A | 24 February 2023 |
| | | | | US | 2022-0371984 | A1 | 24 November 2022 |
| | | | | WO | 2022-108050 | A1 | 27 May 2022 |
| KR | 10-1663586 | B1 | 10 October 2016 | | None | | |
| KR | 10-2022-0041009 | A | 31 March 2022 | CN | 116137860 | A | 19 May 2023 |
| | | | | EP | 4219441 | A1 | 02 August 2023 |
| | | | | JP | 2023-534443 | A | 09 August 2023 |
| | | | | KR | 10-2390132 | B1 | 25 April 2022 |
| | | | | US | 2023-0303476 | A1 | 28 September 2023 |
| | | | | WO | 2022-065852 | A1 | 31 March 2022 |
| KR | 10-2014-0071536 | A | 12 June 2014 | KR | 10-2014-0077342 | A | 24 June 2014 |
| | | | | US | 2014-0142272 | A1 | 22 May 2014 |
| | | | | US | 9527953 | B2 | 27 December 2016 |
| US | 2010-0137631 | A1 | 03 June 2010 | CN | 101631761 | A | 20 January 2010 |
| | | | | CN | 101631761 | B | 19 December 2012 |
| | | | | EP | 2121560 | A1 | 25 November 2009 |
| | | | | EP | 2121560 | B1 | 20 July 2011 |
| | | | | JP | 2010-520944 | A | 17 June 2010 |
| | | | | JP | 2014-088370 | A | 15 May 2014 |
| | | | | JP | 5830507 | B2 | 09 December 2015 |
| | | | | KR | 10-1506046 | B1 | 25 March 2015 |
| | | | | KR | 10-2009-0130042 | A | 17 December 2009 |
| | | | | US | 8344174 | B2 | 01 January 2013 |
| | | | | WO | 2008-110306 | A1 | 18 September 2008 |

Form PCT/ISA/210 (patent family annex) (July 2022)

**EP 4 600 241 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020220126487